# FASCICULE DE BREVET EUROPEEN

(11) **EP 4 007 521 B1**
(45) Date de publication et mention de la délivrance du brevet: **01.07.2026**
(21) Numéro de dépôt: 20767821.0
(22) Date de dépôt: 09.09.2020
(51) Int. Cl.: A61B 5/24, A61B 5/00

(54) **DISPOSITIF DE DÉTERMINATION DU POTENTIEL ÉLECTRIQUE DU CERVEAU**
VORRICHTUNG ZUM BESTIMMEN DES ELEKTRISCHEN POTENZIALS DES GEHIRNS
DEVICE FOR DETERMINING THE ELECTRIC POTENTIAL OF THE BRAIN

(30) Priorité: 09.09.2019 FR 1909895
(43) Date de publication de la demande: 08.06.2022
(73) Titulaire: NAOX Technologies, 91120 Palaiseau (FR)
(72) Inventeur: HUNG-CUONG DINH, Hugo, 91120 PALAISEAU (FR); KABABE, Khalil, 60000 BEAUVAIS (FR); LANGE, Emmanuel, 75015 PARIS (FR)
(74) Mandataire: Loyer & Abello
(86) Numéro de dépôt international: PCT/EP2020/075253
(87) Numéro de publication internationale: WO 2021/048239

(56) Documents cités:
- US-A- 2 798 210
- US-A1- 2018 235 540
- US-A1- 2019 192 077

## Description

### Domaine technique

L'invention se rapporte au domaine des dispositifs pour déterminer un état physiologique ou psychique d'un mammifère, en particulier les dispositifs pour déterminer un tel état par mesure d'un signal électroencéphalogramme (EEG).

### Arrière-plan technologique

On connait un dispositif intra-auriculaire de mesure de données biologiques d'un homme, tel que celui décrit par US 2018/0235540. Ce dispositif comprend une partie interchangeable prévue pour être insérée dans un canal auditif munie sur sa surface extérieure d'électrodes prévues pour détecter un signal électrique en provenance du cœur. Ce dispositif comprend aussi un boitier, sur lequel est monté une électrode externe destinée à accueillir un doigt de l'utilisateur pour former un potentiel de référence.

Le remplacement de la partie interchangeable de ces dispositifs est contraignant et complexe à réaliser. Enfin, la détection de signaux électriques par les électrodes peut manquer de précision.

US2798210A divulgue un assemblage d'anneau glissant pour la transmission d'un courant électrique entre des composants rotatifs. US2019192077A1 divulgue un système de détection pour détecter des signaux électriques conforme au préambule de la revendication 1.

### Résumé

Une idée à la base de cette invention consiste à fournir un dispositif de mesure de données biologiques plus facile à exploiter et plus susceptible de fournir des signaux plus précis.

Pour cela, l'invention propose un dispositif permettant par exemple de déterminer un état physiologique ou psychique d'un mammifère.

Un dispositif selon l'invention est conforme à la revendication indépendante 1 ou à la revendication indépendante 6.

Selon des modes de réalisation avantageux, un tel dispositif peut présenter une ou plusieurs des caractéristiques suivantes.

Selon l'invention, chacune des premières pistes électriques, ou chacune des deuxièmes pistes électriques, présente une forme annulaire ayant pour axe ledit axe de révolution et est agencée en contact électrique avec une desdites premières pistes électriques, ou une desdites deuxièmes pistes électriques, indépendamment de l'orientation de l'embout autour dudit axe de révolution, et lesdites premières ou deuxièmes pistes électriques de forme annulaire étant espacées le long dudit axe de révolution.

Un tel dispositif est avantageux en ce qu'il permet de retirer l'embout et de le remplacer par un autre embout dans une orientation circonférentielle quelconque sans nécessiter des moyens de guidage pour assurer la liaison entre les électrodes et le moyen de calcul. Le dispositif est ainsi moins complexe à exploiter. Un autre avantage de l'utilisation des pistes circulaires est de permettre une rotation des électrodes autour de l'axe de révolution. L'orientation de l'embout peut donc être facilement ajustée, si besoin, pour qu'une ou plusieurs des électrodes soit orientée vers une zone spécifique située autour du canal auditif. Les signaux captés par les électrodes peuvent donc être plus ciblés et permettre une détermination plus précise de l'état physiologique ou psychique du mammifère.

Les électrodes du dispositif peuvent être réalisées de nombreuses manières. Selon un mode de réalisation, au moins une électrode présente une partie externe s'étendant sur une partie longitudinale, dans la direction de l'axe de révolution, de la surface extérieure de l'embout. Selon un mode de réalisation, les électrodes sont espacées mutuellement dans une direction circonférentielle autour de l'axe de révolution.

Selon un mode de réalisation, au moins une électrode présente une longueur égale ou légèrement inférieure à la longueur de l'embout.

Selon un mode de réalisation au moins une électrode présente une partie interne s'étendant sur une partie du canal cylindrique pour former une première piste électrique.

Selon un mode de réalisation, les électrodes sont espacées mutuellement dans une direction circonférentielle par une distance constante.

Selon un mode de réalisation, chacune des deuxièmes pistes électriques est annulaire, et chacune des premières pistes électriques est configurée pour entrer en contact avec une deuxième piste électrique annulaire respective. Selon un mode de réalisation, les premières pistes électriques s'étendent en arc de cercle sur la partie intérieure du canal cylindrique autour de l'axe de révolution. Selon un mode de réalisation, les premières pistes électriques constituent un dépôt métallique sur le canal cylindrique de l'embout.

Selon un autre mode de réalisation, chacune des premières pistes électriques est annulaire et chacune des deuxièmes pistes électriques forme un plot de contact configuré pour entrer en contact avec une première piste électrique annulaire respective. Selon un mode de réalisation, le plot de contact s'étend sur un arc de cercle de la partie cylindrique du corps principal autour de l'axe de révolution. Selon un mode de réalisation alternatif, le plot de contact a une forme rectangulaire, demi-sphérique, annulaire ou semi-annulaire. Selon un mode de réalisation, le plot de contact est un dépôt métallique sur la surface extérieure de la partie cylindrique ou du canal de l'embout.

Les embouts du dispositif peuvent être réalisés selon différentes configurations. Selon un mode de réalisation, l'embout est en matériau élastique configuré pour s'adapter à une dimension, en particulier une circonférence, du canal auditif de manière à assurer un contact entre les électrodes et la surface interne du canal auditif.

Selon un mode de réalisation, l'embout comporte une matière polymère et au moins une électrode comporte un tissu conducteur incrusté ou noyé dans la matière polymère de l'embout.

Selon un mode de réalisation alternatif, l'embout comporte une matière polymère et au moins une électrode comporte un matériau conducteur sélectionné parmi les polymères conducteurs et le silicone dopé de nanoparticules, incrusté ou noyé dans la matière polymère de l'embout.

Selon un autre mode de réalisation, l'embout comporte une matière polymère comprenant une pluralité de parties conductrices isolées électriquement entre elles et formant les électrodes.

Selon un mode de réalisation, le dispositif comprend des moyens de verrouillage de l'embout sur la partie cylindrique du corps principal.

Selon un mode de réalisation, ces moyens de verrouillage comprennent une rainure et/ou une nervure et/ou une butée agencée dans la partie cylindrique du corps principal et/ou dans l'embout.

Selon un mode de réalisation, le dispositif comporte des moyens de traitement incluant un appareil de mesure permettant de mesurer des quantités physiques sur les signaux électriques captés par l'électrode de référence et l'au moins une électrode de mesure. Ces quantités physiques sont par exemple l'intensité ou la tension des signaux captés par les électrodes.

Selon un mode de réalisation, les moyens de traitement sont configurés pour amplifier les signaux électriques captés par les électrodes avant transmission à l'appareil de mesure. Selon un mode de réalisation, cette amplification est réalisée au moyen d'un adaptateur d'impédance.

Selon l'invention, le moyen de calcul est configuré pour déterminer l'état physiologique ou psychique en fonction d'au moins une différence entre le signal électrique capté par l'une des électrodes de mesure et le signal électrique capté par l'électrode de référence. Selon un mode de réalisation, les éventuels artefacts présents sur ces signaux sont supprimés grâce à une méthode de suppression d'artefacts à plusieurs canaux, par exemple l'analyse en composantes indépendantes. Selon un mode de réalisation, la ou les différences entre le signal électrique capté par l'une des électrodes de mesure et le signal électrique capté par l'électrode de référence est quantifiée sous la forme d'une différence de potentiel. Selon un mode de réalisation, le moyen de calcul détermine cette différence de potentiel à partir de la tension des signaux mesurée par l'appareil de mesure.

Selon un mode de réalisation, l'appareil de mesure permet de mesurer des quantités physiques sur le signal électrique capté par l'électrode de référence grâce à au moins une première résistance dans laquelle circule un courant correspondant à une somme entre un courant correspondant au signal électrique capté par l'électrode de référence et un courant provenant d'un générateur de référence présentant des caractéristiques connues. Selon un mode de réalisation, l'au moins une résistance peut être une résistance seule ou un montage électrique constitué de plusieurs résistances. Selon un mode de réalisation, le montage électrique de plusieurs résistances peut être un montage de résistances en série. Les caractéristiques physiques du signal électrique capté par l'électrode de référence peuvent être déterminées en appliquant la loi d'Ohm à la résistance si elle est unique, ou en appliquant une formule de pont diviseur de tension si on considère un montage électrique de résistances. Dans ce mode de réalisation, la valeur de la résistance ou des résistances utilisées est connue.

Dans un mode de réalisation, le générateur est un générateur de courant. Dans un mode de réalisation alternatif, le générateur est un générateur de tension. Les caractéristiques physiques du signal électrique capté par l'électrode de référence peuvent être déterminées en appliquant la loi d'Ohm à la résistance. Dans un mode de réalisation, le générateur alimentant la résistance délivre un courant de tension considérablement élevée (en valeur absolue) par rapport aux variations de tension attendues dans le signal capté par l'électrode de référence. Un avantage de ce mode de réalisation est de pouvoir détecter la présence de valeurs aberrantes. Ce mode de réalisation est avantageux notamment pour calculer une différence de potentiel entre un signal capté par une électrode de mesure et un signal capté par l'électrode de référence selon le mode de réalisation ci-dessous.

Selon un mode de réalisation avantageux, l'appareil de mesure permet de mesurer des quantités physiques sur le signal électrique capté par l'au moins une électrode de mesure grâce à au moins une deuxième résistance dans laquelle circule un courant correspondant à une somme entre un courant correspondant au signal électrique capté par l'au moins une électrode de mesure et un courant provenant d'un second générateur présentant des caractéristiques connues et délivrant une tension supérieure à celle délivrée par le générateur de référence. Selon un mode de réalisation, l'au moins une résistance peut être une résistance seule ou un montage électrique constitué de plusieurs résistances. Selon un mode de réalisation, le montage électrique de plusieurs résistances peut être un montage de résistances en série. Les caractéristiques physiques du signal électrique capté par l'électrode de référence peuvent être déterminées en appliquant la loi d'Ohm à la résistance si elle est unique, ou en appliquant une formule de pont diviseur de tension si on considère un montage électrique de résistances. Selon un mode de réalisation, la tension délivrée par le second générateur est positive et de valeur considérablement plus élevée que les variations de tension attendues dans le signal provenant d'une électrode de mesure.

Ce mode de réalisation avantageux permet de repérer facilement les mesures aberrantes. En effet, dans ce mode de réalisation, il est attendu que la différence de potentiel entre la tension du signal mesuré par une électrode de mesure et la tension du signal mesuré par l'électrode de référence fluctue peu autour de la demie-somme de la tension alimentant l'au moins une résistance permettant de mesurer la tension du signal capté par l'électrode de mesure et de la tension alimentant l'au moins une résistance permettant de mesurer la tension du signal capté par l'électrode de référence. Par exemple, si la résistance (ou le montage de résistances en série) permettant de mesurer la tension du signal capté par l'électrode de mesure est alimentée par une tension de 2,4 V et si la résistance (ou le montage de résistances en série) permettant de mesurer la tension du signal capté par l'électrode de mesure est alimentée par une tension proche de 0 V (par exemple de l'ordre de 0,1 V), et que la fluctuation de tension dans le signal capté par les électrodes est attendue autour du µV, la différence de potentiel obtenue doit être autour de 1,2V, avec des fluctuations de l'ordre du µV. Toute mesure qui s'écarte significativement de cette valeur, par exemple de quelques mV, pourra être considérée comme une valeur aberrante.

Selon un mode de réalisation, l'embout est constitué de sorte à pouvoir être en contact avec la partie spécifique du canal auditif lorsqu'il est inséré dans le canal auditif, et l'électrode de masse est située sur une partie de l'embout en contact avec la partie spécifique du canal auditif afin de de capter un signal électrique sur ladite partie spécifique du canal auditif. Selon un mode de réalisation, la partie spécifique du canal auditif est un tragus du mammifère. Dans le cas d'un humain, le tragus correspond à un cartilage de l'oreille situé à l'entrée du canal auditif.

Selon un mode de réalisation, la réduction de bruit est réalisée au moyen d'un circuit de réjection de mode commun. Ce mode de réalisation est avantageux car il permet de réduire le bruit provenant de perturbations extérieures au corps du mammifère.

Dans le dispositif selon la revendication 6, l'électrode de masse est branchée sur un potentiel correspondant à une demie-somme des tensions délivrées par deux générateurs de tension, par exemple les deux générateurs de l'appareil de mesure. Ce mode de réalisation est avantageux, car il permet de définir identiquement un potentiel nul dans tout le circuit.

Selon un mode de réalisation, les moyens de traitement comportent une première résistance branchée sur un premier générateur de tension et agencée de manière à recevoir le signal électrique capté par l'électrode de référence et une deuxième résistance branchée sur un deuxième générateur de tension et agencée de manière à recevoir le signal électrique capté par l'au moins une électrode de mesure, le deuxième générateur de tension délivrant une tension supérieure à celle délivrée par le premier générateur de tension.

Dans le mode de réalisation faisant intervenir une électrode de masse, l'électrode de référence et les électrodes de mesure peuvent être choisies parmi les électrodes autres que l'électrode de masse.

Selon un mode de réalisation, en particulier en l'absence d'une électrode de masse, un des signaux électriques est un signal électrique de référence ; de préférence il s'agit d'un signal électrique substantiellement stable. En d'autres termes, l'électrode de référence est définie comme celle captant un signal électrique substantiellement stable.

Selon un mode de réalisation, le signal électrique de référence est mesuré par une électrode, à savoir l'électrode de référence, laquelle est destinée à être orientée vers une partie osseuse à proximité du canal auditif du mammifère. En particulier, cette partie osseuse est une mastoïde.

Un avantage d'un tel agencement est d'obtenir un signal de référence stable, c'est-à-dire qui fluctue peu, sans nécessiter de recourir à une électrode externe à l'embout.

Selon un mode de réalisation, l'embout comporte un marquage configuré pour reconnaître visuellement l'électrode de référence. Selon un mode de réalisation l'électrode de référence est branchée sur un potentiel prédéfini.

Selon un mode de réalisation le potentiel prédéfini correspond à une demie-somme des tensions délivrées par les deux générateurs de tension précités. Selon un mode de réalisation, l'électrode de référence ainsi branchée est placée sur l'embout de manière à être en contact avec la surface du canal auditif du mammifère. Ce mode de réalisation est avantageux car il permet d'électrifier artificiellement la peau en lui transmettant le potentiel sur lequel est branchée l'électrode de référence (puisque l'électrode de référence est branchée sur un certain potentiel et est également collée à la surface du canal auditif). Cela permet d'augmenter la stabilité du signal capté par l'électrode de référence à la surface du canal auditif où elle est collée. En effet, il est alors attendu que le signal provenant de cette partie du canal auditif fluctue légèrement autour de ce potentiel, en étant considérablement stable.

Selon des modes de réalisation, l'embout comporte des moyens de détermination du signal électrique de référence. Selon un mode de réalisation, l'embout comporte un marquage, de préférence sur sa surface extérieure, configuré pour être dirigé vers la mastoïde du mammifère lors de l'insertion de l'embout dans le canal auditif du mammifère, de sorte qu'une des électrodes est dirigée vers la mastoïde du mammifère. En d'autres termes, ce marquage indique comment orienter l'une des électrodes vers une partie osseuse à proximité du canal auditif du mammifère, en particulier, la mastoïde.

Selon un mode de réalisation alternatif, le signal de référence est détecté automatiquement grâce à un sélectionneur. En particulier, le sélectionneur est configuré pour recevoir les signaux électriques des électrodes et pour détecter une électrode de référence, ladite électrode de référence étant sélectionnée comme l'électrode émettant le signal électrique ayant le potentiel électrique le plus stable, c'est-à-dire celui présentant le moins de fluctuations, parmi les signaux électriques captés par les électrodes.

Selon un mode de réalisation, le sélectionneur de signal est agencé en amont du moyen de calcul et relié aux deuxièmes pistes électriques. Selon un mode de réalisation, le sélectionneur est relié à un amplificateur comportant une entrée négative et au moins une entrée positive, et dans lequel le signal sélectionné par le sélectionneur est acheminé vers l'entrée négative de l'amplificateur. En particulier, l'amplificateur est relié au moyen de calcul.

Selon un mode de réalisation, le sélectionneur est constitué d'un mécanisme de branchement, par exemple un connecteur réversible agencé en amont de l'amplificateur et d'un testeur de signal agencé en aval dudit amplificateur. Le connecteur réversible peut être apte à connecter chacune des deuxièmes pistes électriques à une entrée de l'amplificateur et le testeur de signal peut être configuré pour déterminer un signe de la ou les différences entre le signal provenant de l'au moins une entrée positive et le signal provenant de l'entrée négative. Le sélectionneur peut être configuré pour acheminer les signaux en sortie de l'amplificateur vers le moyen de calcul en réponse à la détection par le testeur de signal que toutes les différences sont positives. Le sélectionneur peut être en outre configuré à modifier une configuration du connecteur réversible de sorte à connecter une autre des deuxièmes pistes à l'entrée négative de l'amplificateur en réponse à la détection par le testeur de signal d'au moins différence négative parmi des différences calculées. Dans ce mode de réalisation, le signal électrique le plus stable est celui ayant le potentiel électrique le plus bas.

Selon un mode de réalisation, au moins une des électrodes est orientée vers un lobe temporal du mammifère.

Selon un mode de réalisation, l'état physiologique ou psychique est relatif à une activité électrique d'un organe du mammifère sélectionné dans le groupe constitué d'un cerveau, un coeur, un nerf, un muscle et un œil.

Selon un mode de réalisation, l'état physiologique ou psychique est relatif à un signal EEG déterminé par le moyen de calcul à partir d'au moins un signal électrique émis par une zone du cerveau du mammifère et capté par une ou plusieurs des électrodes agencées dans le canal auditif.

Selon un mode de réalisation, l'état physiologique ou psychique est une fatigue du mammifère, un niveau d'attention du mammifère ou une crise d'épilepsie du mammifère.

Selon un mode de réalisation, l'état psychique est une activité pré-ictale du cerveau.

Selon un mode de réalisation, le dispositif comprend un moyen de filtrage des signaux électriques configuré pour atténuer des signaux parasites détectés par une desdites électrodes
De tels signaux parasites peuvent être engendrés par des mouvement du mammifère et/ou de l'embout dans le canal auditif et/ou provenir de l'environnement du dispositif.

Selon un mode de réalisation, le moyen de filtrage comprend un filtre passe bande ayant une bande passante comprise entre 0,3 Hz et 50 Hz, en particulier comprise entre 1 Hz et 40 Hz.

Selon un mode de réalisation, le moyen de calcul est configuré pour :
déterminer un signal d'électroencéphalogramme du mammifère en fonction des signaux électriques mesurés,
déterminer une amplitude d'au moins une onde cérébrale dans une plage de fréquence prédéfinie en fonction dudit signal d'électroencéphalogramme, et
déterminer un état psychique en fonction de ladite amplitude d'au moins une onde cérébrale par comparaison de ladite amplitude à un seuil prédéterminé.

Une application du dispositif est la détection d'une somnolence d'une personne et l'utilisation de cette détection pour déclencher un réveil de la personne.

Selon un mode de réalisation, le corps principal comporte un moyen de transmission de données filaire ou sans fil configuré pour communiquer avec le moyen de calcul. Le moyen de transmission est configuré pour transmettre les signaux électriques captés par les électrodes au moyen de calcul.

Selon un mode de réalisation, le corps principal est un boitier isolant électriquement comprenant le moyen de transmission de données vers le moyen de calcul et les moyens de traitement du signal.

Selon un mode de réalisation, le dispositif comprend une seule oreillette.

Selon un mode de réalisation alternatif, le dispositif comprend deux oreillettes. Les oreillettes sont identiques ou différentes.

Selon un mode de réalisation correspondant, le dispositif comprend deux oreillettes configurées pour être agencées respectivement dans un premier canal auditif du mammifère et un deuxième canal auditif du mammifère et destinées à acheminer des signaux électriques respectifs vers un moyen de calcul. En particulier, le moyen de calcul est destiné à déterminer l'état physiologique ou psychique en fonction d'un signal mesuré par une électrode de l'oreillette agencée dans le premier canal auditif et un signal mesuré par une électrode de l'oreillette agencée dans le deuxième canal auditif, notamment en fonction d'une différence entre les deux signaux.

Ainsi dans un mode de réalisation, au moins une différence entre deux signaux captés respectivement dans le canal auditif d'une oreille gauche et d'une oreille droite du mammifère est exploitée.

Selon un mode de réalisation, le moyen de calcul est agencé à distance du corps principal.

Selon un mode de réalisation correspondant, le dispositif comprend un seul moyen de calcul.

Selon un autre mode de réalisation, le moyen de calcul est agencé dans le corps principal d'une oreillette.

Selon un mode de réalisation correspondant, chaque oreillette comprend un moyen de calcul.

### Brève description des figures

L'invention sera mieux comprise, et d'autres buts, détails, caractéristiques et avantages de celle-ci apparaîtront plus clairement au cours de la description suivante de plusieurs modes de réalisation particuliers de l'invention, donnés uniquement à titre illustratif et non limitatif, en référence aux dessins annexés.
[fig.1] la figure 1 est une vue de côté d'un dispositif selon un premier mode de réalisation de l'invention.
[fig.2] la figure 2 est une vue de l'intérieur du dispositif dans le plan de la figure 1.
[fig. 3] Les figures 3a et 3b sont respectivement une vue de dessus et une vue tridimensionnelle en coupe de la partie supérieure d'un embout de la figure 3a et pouvant être utilisé dans le dispositif de la figure 1.
[fig. 4] La figure 4 est une vue en coupe d'un dispositif selon un deuxième mode de réalisation de l'invention.
[fig. 5] la figure 5 est un schéma représentatif de moyens de traitement d'un signal électrique pouvant être utilisés dans un dispositif selon un mode de réalisation de l'invention.
[fig. 6] la figure 6 est un schéma représentatif d'un dispositif selon un troisième mode de réalisation de l'invention.
[fig. 7] la figure 7 est un schéma représentatif d'un procédé de traitement des données pouvant être mis en œuvre par un moyen de calcul du dispositif selon un mode de réalisation de l'invention.
[fig. 8] Les figures 8a et 8b sont des schémas représentatifs de deux modes de réalisation d'un sélectionneur automatique de signal.
[fig. 9] La figure 9 est une vue en perspective d'un embout comportant des électrodes selon un autre mode de réalisation.
[fig. 10] La figure 10est une vue de dessus de l'embout de la figue 9.
[fig. 11] La figure 11 est un schéma fonctionnel électrique d'une oreillette selon un mode de réalisation reliée au cerveau du mammifère.
[fig. 12] La figure 12 est un schéma fonctionnel électrique d'une oreillette selon un autre mode de réalisation reliée au cerveau du mammifère.

### Description des modes de réalisation

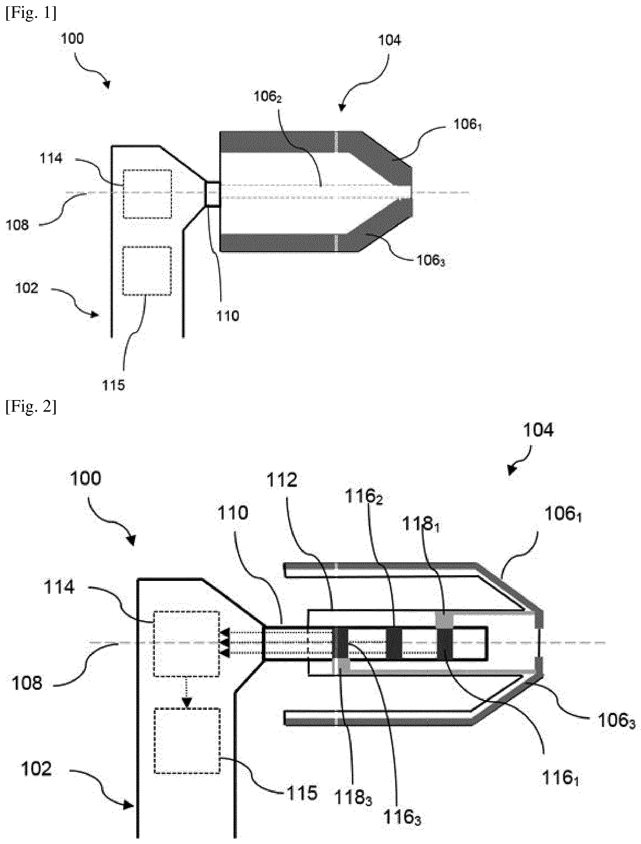

Sur les figures 1 et 2, un premier mode de réalisation d'un dispositif pour déterminer un état psychique ou physiologique d'un mammifère, en particulier une personne, est représenté.

Le dispositif 100 est prévu pour déterminer des données d'électroencéphalogramme (EEG) d'une personne. Le dispositif 100 comprend une oreillette, par exemple de forme et de taille semblable à un écouteur, comprenant un boitier 102 et un embout 104 amovible.

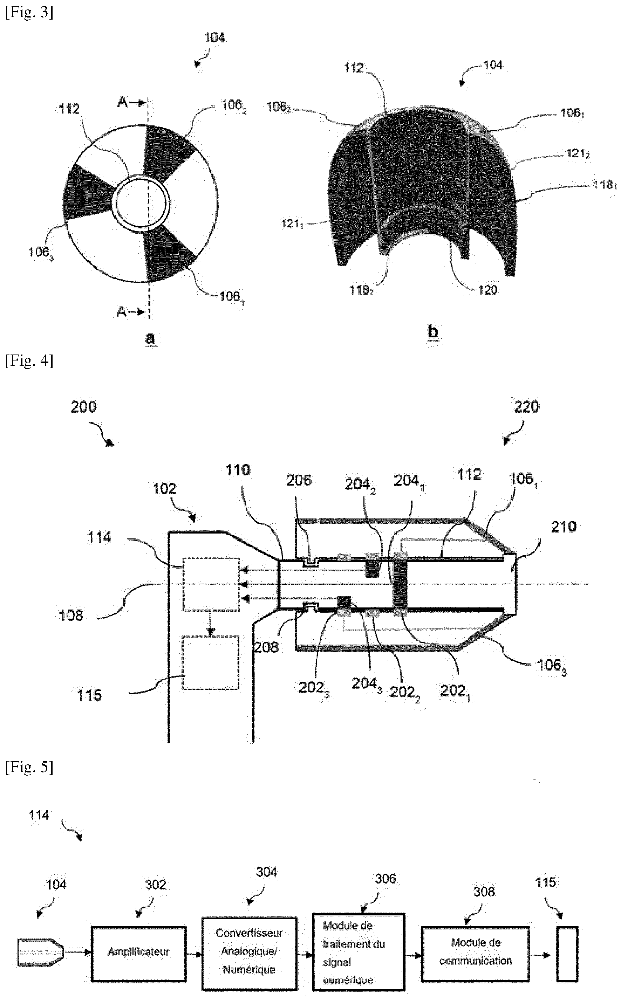

Dans la figure 1, le dispositif est vu de côté, donc les parties situées à l'extérieur de celui-ci sont visibles, des parties intérieures ou non visibles étant représentées en pointillés. Dans la figure 2, l'embout 104 est représenté en coupe dans le plan de la figure 1, tandis que les autres éléments sont représentés vus de côté. Les éléments situés à l'intérieur du boîtier 102 sont représentés en pointillés. L'embout 104 comprend une partie bombée et un canal cylindrique 112. La partie bombée est rabattue par-dessus un canal cylindrique 112, qui n'est solidaire de la partie bombée que dans la partie supérieure, comme représenté sur la figure 3b. Ainsi, un espace vide se trouve entre le canal cylindrique 112 et la surface extérieure de l'embout 104.

L'embout 104 est muni sur sa surface extérieure de trois électrodes 106₁, 106₂ et 106₃ configurées pour capter des signaux électriques dans un canal auditif de la personne. L'embout 104 est une pièce de révolution autour d'un axe de révolution 108 et les électrodes sont disposées équidistantes l'une de l'autre dans une direction circonférentielle de l'embout 104. L'embout 104 est en matière isolante, par exemple en silicone et les électrodes 106 sont par exemple des morceaux de tissus conducteurs incrustés dans le silicone de l'embout 104, par exemple par collage.

Les dimensions de l'embout 104 sont configurées pour s'adapter au canal auditif. De plus, l'embout 104 étant en silicone, les électrodes 106 sont maintenues en contact avec une paroi intérieure du canal auditif grâce à l'élasticité de la silicone. L'embout 104 présente une longueur comprise entre 15 mm et 25 mm et un diamètre supérieur au diamètre du canal auditif, en particulier d'un pourcentage compris entre 2% et 5% du diamètre du canal auditif.

Le boitier 102 comprend des moyens de traitement 114 configurés pour recevoir les signaux électriques captés par les électrodes 106, traiter ces signaux et les transformer en signaux numériques. Les moyens de traitement 114 sont reliés à un moyen de calcul 115, qui peut être intégré au boitier 102 ou séparé de celui-ci, pour déterminer en fonction des signaux traités par les moyens de traitement 114, des données d'EEG. Dans un autre mode de réalisation, les moyens de traitement 114 peuvent aussi être agencés à l'extérieur du boitier 102.

Le boitier 102 comprend aussi un arbre 110 s'étendant du boitier 102 et ayant pour axe l'axe de révolution 108. L'arbre 110 est configuré pour recevoir le canal cylindrique 112 formé dans l'embout 104. L'arbre 110 est muni de trois pistes électriques annulaires 116₁, 116₂ et 116₃ respectivement reliées aux électrodes 106₁, 106₂ et 106₃. Les pistes annulaires 116 ont pour axe l'axe de révolution 108 et sont agencées à distance l'une de l'autre dans la direction de l'axe de révolution 108. L'arbre 110 est en matériau isolant et les pistes électriques 116 sont formées par un dépôt métallique sur l'arbre 110. Les pistes annulaires 116 sont reliées aux moyens de traitement 114 par des fils électriques agencés à l'intérieur de l'arbre 110. Chacune des pistes annulaires 116₁ et 116₃ est reliée à une électrode 106, et106₃ par des premières pistes électriques 118₁ et 118₃ (la piste électrique reliant l'électrode 106₂ à la piste annulaire 116₂ n'est pas représentée sur les figures 1 et 2).

Sur les figures 3a et 3b, l'embout 104 est, respectivement, représenté selon une vue de dessus et une vue tridimensionnelle suivant une coupe A-A.

Les électrodes 106 s'étendent sur toute la longueur de l'embout 104 dans la direction de l'axe de révolution 108. De plus, les électrodes 106 sont distribuées sur la surface extérieure de l'embout 104 de façon équidistante autour de l'axe de révolution 108. En particulier, les électrodes 106 s'étendent sur une surface extérieure de l'embout d'environ 75 % comprise entre 50% et 80 % de la surface totale extérieure de l'embout 104. D'autres distributions de la surface des électrodes seraient aussi possibles.

Les premières pistes électriques 118 sont agencées dans l'embout 104 selon un mode de réalisation représenté sur la figure 3b. La partie supérieure de l'embout 104 y a été représentée coupée selon l'axe A de la figure 3a, donc seules deux électrodes 106₁ et 106₂ et deux premières pistes électriques 118₁ et 118₂ ont été représentées. L'embout 104 représenté sur cette figure est réalisé grâce à un moule à tiroirs, qui permet d'obtenir trois parties creuses sur la surface bombée de l'embout 104, destinées à recevoir les électrodes 106, ces parties creuses s'étendant chacune sous la forme d'un puits initialement vide à l'intérieur de la partie isolante de l'embout 104. Sur la figure 3b ont été représentés les puits 121₁ et 121₂, composés d'une portion verticale d'étendant selon une partie longitudinale du canal cylindrique 112 et une portion horizontale s'étendant en arc de cercle, destinés à recevoir les pistes électriques 118₁ et 118₂. Après moulage et solidification de la partie isolante, un matériau conducteur liquide est coulé dans chacun de ces puits 121, formant ainsi les premières pistes électriques 118₁, 118₂ et 118₃, chaque piste électrique occupant alors un puits 121. Le matériau conducteur liquide est coulé dans les puits jusqu'à ce que ceux-ci débordent pour remplir les creux à la surface de de la partie bombée de l'embout. Le matériau conducteur liquide ayant débordé dans les creux se solidifie ensuite pour former les électrodes 106. Puisqu'il n'y a pas de contact entre les différents creux et les différents puits 121, l'isolation électrique respective des électrodes 106 et des pistes électriques 118 coulées à l'intérieur est assurée.

De plus, le canal cylindrique 112 de l'embout 104 possède sur sa surface intérieure un épaulement 120 représenté sur la figure 3b. Celui-ci sert à maintenir l'embout sur l'arbre 110. Dans un mode de réalisation, cet épaulement 120 peut être absent.

Dans un autre mode de réalisation non représenté sur les figures, les premières pistes électriques 118₁, 118₂ et 118₃ sont des plots de contact reliés aux électrodes 106 par des fils électriques isolés électriquement et incrustés à la surface de la partie bombée de l'embout 104 et sur la surface intérieure du canal cylindrique 112.

En particulier, l'arbre 110 présente un diamètre légèrement inférieur au diamètre du canal cylindrique 112 de l'embout 104, par exemple inférieur d'une valeur comprise entre 0,5 mm et 2 mm.

Les premières pistes électriques 118 étant agencés en regard des pistes annulaires 116, l'embout 104 est susceptible d'être tourné autour de l'axe de révolution 108 sans risque d'interruption de la liaison électrique entre les électrodes 116 et les moyens de traitement 114. De plus, le remplacement de l'embout 104 s'en trouve facilité car il n'est pas nécessaire de recourir à des moyens de guidage pour assurer la liaison électrique lors du montage de l'embout 104 sur l'arbre 110.

La figure 4 montre une vue analogue à la figure 2 selon un deuxième mode de réalisation. A la différence du dispositif 100, le dispositif 200 de la figure 4 comprend un embout plein 220, avec un canal cylindrique 112 creusé à l'intérieur de celui-ci. L'embout plein 220 comprend en outre trois premières pistes électriques annulaires 202₁, 202₂ et 202₃ agencées dans le canal cylindrique 112 et reliées aux électrodes 106₁, 106₂ et 106₃, respectivement, par des fils électriques isolés électriquement entre eux et incrustés dans l'embout 220, entre sa surface et le canal cylindrique 112.

L'arbre 110 est muni de trois pistes électriques non annulaires 204₁, 204₂ et 204₃ espacées entre elles dans la direction de l'axe de révolution 108. Les pistes électriques non annulaires sont formées par un dépôt métallique s'étendant par exemple selon un demi-cercle de l'arbre autour de l'axe de révolution 108 et faisant saillie en direction de l'embout plein 220. Les pistes électriques non annulaires 204 sont agencées en regard des pistes électriques annulaires 202 de sorte à maintenir un contact électrique avec elles.

L'arbre 110 comporte une gorge 208 agencée du côté du boitier 102 et configurée pour recevoir un collet 206 prévu dans le canal cylindrique 112 de l'embout 104. Cet agencement permet de bloquer la translation de l'embout plein 220 dans la direction de l'axe de révolution 108. L'arbre 110 comprend aussi une butée 210 ayant un diamètre supérieur au diamètre de l'arbre 110 de sorte à maintenir l'embout en serrage contre la butée 210 pour stabiliser sa position en rotation.

Sur la figure 5, on a représenté des moyens de traitement 114 qui peuvent être utilisés dans le dispositif 100 ou 200.

Les moyens de traitement 114 sont configurés pour recevoir un ou plusieurs signaux électriques captés par les électrodes 106. Les moyens de traitement 114 comprennent un amplificateur 302 prévu pour amplifier l'amplitude du ou de chaque signal électrique. Le gain de l'amplificateur 302 peut être compris entre 12 fois et 1000 fois. En particulier, les moyens de traitement 114 comprennent un préamplificateur non représenté sur la figure 5. L'amplificateur 302 est relié à un convertisseur analogique/numérique (CAN) 304 configuré pour numériser le ou chaque signal électrique. Le CAN 304 est relié à un module de traitement du signal numérique 306 configuré pour atténuer les éventuels signaux parasites captés par les électrodes. Ces signaux parasites peuvent être dus au mouvement de la tête de la personne, d'un mouvement des fils électriques reliés au dispositif ou à l'environnement du dispositif. Ce module de traitement du signal numérique 306 comporte en particulier un filtre passe-bande, mais aussi des fonctions de traitement du signal, notamment une fonction permettant d'enlever la composante linéaire du signal. Cette composante linéaire est présente du fait de choisir une électrode de référence à l'intérieur du canal auditif sans utiliser une masse située à l'extérieur du canal auditif. La bande passante du filtre passe bande 306 est configurée en outre pour sélectionner des signaux électriques en provenance d'un organe prédéterminé, en particulier le cerveau. Par exemple, la bande passante du filtre passe bande 306 est comprise entre 0,3 Hz et 50 Hz, en particulier comprise entre 1 Hz et 40 Hz. Le filtre passe bande 306 est relié à un module de communication 308 configuré pour transmettre les signaux numérisés par le CAN 304 et filtrés par le filtre passe bande 306 vers le moyen de calcul 115, de façon filaire ou non filaire. Le module de traitement du signal numérique peut aussi comprendre d'autres filtres, par exemple un filtre coupe-bande ou filtre Notch.

Le moyen de calcul 115 du dispositif 100 ou 200 est configuré pour déterminer un signal d'électroencéphalogramme (EEG) en fonction des signaux électriques captés par les électrodes 106, en particulier une différence entre lesdits signaux électriques. Le moyen de calcul 115 est configuré pour déterminer un signal EEG à partir d'une des combinaisons de deux électrodes parmi les électrodes : 106₁, 106₂ et 106₃. L'une des électrodes est choisie comme l'électrode de référence, par rapport à laquelle le potentiel sera calculé. Le calcul du potentiel du cerveau est réalisé en effectuant la différence des signaux captés par les autres électrodes avec le signal capté par l'électrode de référence. Malgré le fait d'avoir effectué un premier traitement du signal numérique pour en éliminer les signaux parasites, par exemple en ayant choisi par l'intermédiaire du filtre décrit précédemment, une fréquence correspondant aux ondes cérébrales, des artefacts peuvent toujours être présents sur les signaux. S'il y en a, ceux-ci sont repérés en comparant les différences des signaux provenant des deux autres électrodes avec l'électrode de référence. En effet, les deux signaux présentent alors les mêmes composantes parasites. En particulier, cette comparaison est réalisée au moyen d'une méthode de suppression d'artefacts à plusieurs canaux, de préférence l'analyse en composantes indépendantes (ou *Independent component analysis*)*.*

Le choix de l'électrode de référence est réalisé en amont du moyen de calcul, selon les trois exemples suivants.

Dans un premier exemple, l'électrode de référence est identifiée au moyen d'un marquage sur l'embout 104, permettant de positionner celle-ci en direction d'une partie osseuse du mammifère par exemple une mastoïde, de manière à optimiser la stabilité du signal, c'est-à-dire en minimiser les fluctuations, pour obtenir un signal substantiellement stable, qui peut alors être utilisé comme signal de référence pour déterminer un signal EEG du mammifère.

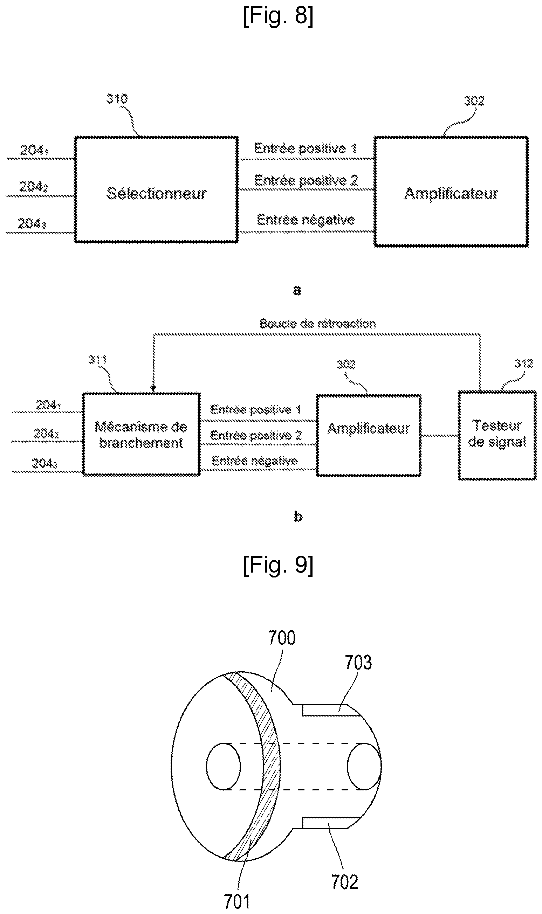

Dans les deux autres exemples, représentés sur les figures 8a et 8b, un sélectionneur automatique de signal permet de déterminer le signal de référence.

Dans un deuxième exemple, le signal de référence est choisi au moyen d'un sélectionneur 310 de signal placé en amont de l'amplificateur 302, ledit sélectionneur 310 sélectionnant le signal le plus stable, c'est-à-dire celui qui présente le moins de fluctuations. L'amplificateur 302 possède une entrée négative et deux entrées positives, l'entrée négative étant destinée à recevoir le signal de référence. Le signal sélectionné comme étant le plus stable est alors acheminé vers l'entrée négative de l'amplificateur 302. Ainsi, le signal le plus stable est identifié et peut être utilisé comme référence pour le calcul du potentiel du cerveau.

Dans un troisième exemple, les deuxièmes pistes électriques 118, 204 sont reliées à l'amplificateur 302 par l'intermédiaire d'un mécanisme de branchement 311, qui permet de choisir la piste électrique à relier à chaque entrée de l'amplificateur 302. L'amplificateur 302 possède, de même que dans l'exemple précédent, une entrée négative et deux entrées positives. Le sélectionneur est constitué d'un mécanisme de branchement 311, par exemple un connecteur réversible, et d'un testeur de signal 312 placé en aval de l'amplificateur 302. Le testeur de signal 312 est configuré pour déterminer les signes des différences de potentiel entre les signaux provenant des entrées positives de l'amplificateur et le signal provenant de l'entrée négative, qui est alors temporairement choisi comme référence. Le signal choisi pour l'entrée négative doit être celui de potentiel le plus bas car il constitue la référence de potentiel. Or, le potentiel le plus bas est supposé coïncider avec le signal le plus stable. Si le testeur de signal 312 détermine que les différences de potentiel entre les signaux provenant des entrées positives de l'amplificateur et le signal provenant de l'entrée négative t sont toutes positives, le signal choisi pour l'entrée négative est bien le plus stable et les signaux en sortie de l'amplificateur peuvent alors être acheminés vers le moyen de calcul 115 (passant d'abord par le reste des moyens de traitement 114). Le signal provenant de l'entrée négative de l'amplificateur sera choisi comme référence de potentiel. Sinon, si au moins l'une des différences est négative, cela signifie que le signal choisi pour l'entrée négative n'était pas celui de potentiel le plus bas. Dans ce cas, une boucle de rétroaction envoie un signal vers le mécanisme de branchement 311, qui modifie les branchements des entrées de l'amplificateur 302, puis le test est de nouveau effectué par le testeur de signal. Les branchements sont ainsi modifiés et le test effectué jusqu'à ce que la bonne configuration, c'est-à-dire celle dans laquelle les différences calculées sont toutes positives, soit trouvée.

Par exemple, si l'électrode choisie comme référence, de manière manuelle, selon le premier exemple exposé ci-dessus, ou automatique, selon l'un des deuxième ou troisième exemples exposés ci-dessous, est l'électrode 106₃, le signal EEG peut ainsi être obtenu à partir de signaux détectés par les électrodes 106₁ et 106₃ ou les électrodes 106₂ et 106₃.

L'oreillette décrite ci-dessus peut être utilisée seule ou en combinaison avec une deuxième oreillette.

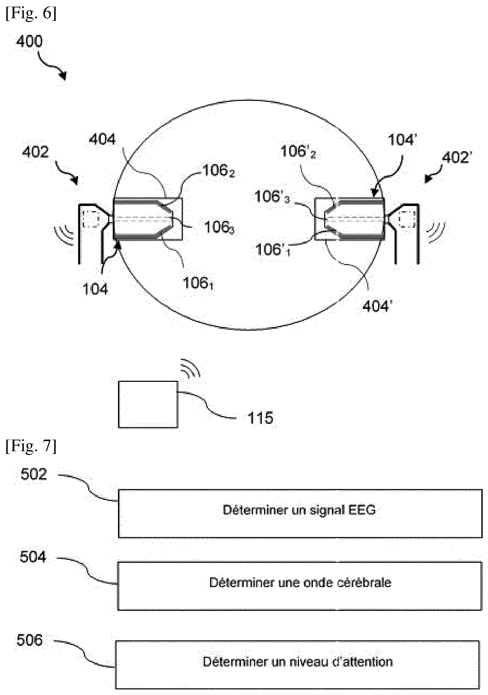

Ainsi, le dispositif 400 de la figure 6 comprend deux oreillettes 402 et 402', similaires à l'oreillette du dispositif 100 ou 200. Chaque oreillette 402 et 402' est munie d'un embout 104 et 104' analogue à l'embout du dispositif 100 ou 200. Chaque embout 104 et 104' est agencé dans un canal auditif 404 et 404' de la personne portant le dispositif 400. A la différence des dispositifs 100 et 200, le moyen de calcul 115 est agencé à distance des oreillettes 402 et les moyens de traitement 114 transmettent les signaux électriques reçus par les électrodes 106 au moyen de calcul 115 de façon non filaire. Le moyen de calcul 115 peut aussi être incorporé à l'une des deux oreillettes.

Les embouts 104 sont en matériau élastique et dimensionnés de sorte à assurer un contact entre la paroi des canaux auditifs et des électrodes 106 et 106' respectivement des embouts 104 et 104'.

Le moyen de calcul 115 est configuré pour déterminer un signal d'électroencéphalogramme (EEG) en fonction des signaux électriques captés par une combinaison de deux électrodes choisies parmi les combinaisons suivantes : 106₁ et 106'₃, 106₂ et 106'₃, 106₃ et 106'₃, 106'₁ et 106'₃, 106'₂ et 106'₃. En particulier, le signal EEG est déterminé par une différence entre les deux signaux en combinaison. De même que dans le cas du fonctionnement à une oreillette, les artefacts encore présents dans le signal sont supprimés au moyen d'une méthode de suppression d'artefacts à plusieurs canaux, de préférence par l'analyse en composantes indépendantes.

Dans ce mode de réalisation à deux oreillettes, l'électrode de référence est choisie selon l'un des exemples présentés dans le mode de fonctionnement à une oreillette. Par exemple, si les électrodes de référence choisies sont 106₃ et 106'3, le signal EEG peut ainsi être obtenu à partir de signaux détectés par une des combinaisons d'électrodes suivantes : 106₁ et 106₃, 106₂ et 106₃, 106'₁ et 106'₃, 106'₂ et 106'₃, 106₁ et 106'₃, 106₂ et 106'₃, 106'₁ et 106₃, et 106'₂ et 106₃.

La figure 7 représente les étapes effectuées par le moyen de calcul 115 du dispositif 100, 200 ou 400 pour déterminer un niveau d'attention de la personne portant le dispositif 100, 200 ou 400.

Le moyen de calcul 115 est configuré pour réaliser :
une étape 502 pour déterminer un signal EEG en fonction de la différence entre deux signaux captés par deux électrodes d'une même oreillette ou de deux oreillettes,
une étape 504 pour déterminer une onde cérébrale en fonction de la fréquence du signal EEG. A titre d'exemple, si la fréquence du signal est comprise est comprise entre 5 Hz et 15 Hz, en particulier égale à 10 Hz, l'onde cérébrale est du type α et si la fréquence du signal est comprise entre 15 Hz et 25 Hz, en particulier égale à 20 Hz, l'onde cérébrale est du type β. L'onde cérébrale peut être déterminée par tout moyen de traitement de signal, en particulier par application d'une transformée de Fourier et
une étape 506 pour déterminer un niveau d'attention en fonction de l'onde cérébrale. En particulier, le niveau d'attention est déterminé en fonction de l'amplitude de l'onde cérébrale ou un du rapport entre deux amplitudes de deux ondes cérébrales. A titre d'exemple, pour une onde cérébrale du type α, le moyen de calcul 115 détermine que la personne manque d'attention pour une amplitude de l'onde α inférieure à un premier seuil prédéterminé. Pour une onde cérébrale du type β, le moyen de calcul 115 détermine que la personne manque d'attention pour une amplitude de l'onde β inférieure à un deuxième seuil prédéterminé. En particulier, le moyen de calcul 115 détermine le niveau d'attention par comparaison du rapport d'amplitudes des ondes α et β à un troisième seuil prédéterminé.

Dans un mode de réalisation, le moyen de calcul 115 peut comprendre une étape de déclenchement d'un signal de réveil destiné à réveiller la personne portant le dispositif 100, 200 ou 400.

En outre, lors du remplacement de l'embout 104, le montage du nouvel embout 104 peut être selon une orientation indifférente ou prédéterminée autour de l'axe de révolution. La liaison électrique entre les électrodes 106 et le moyen de calcul 115 est assurée par les pistes annulaires 118 ou 202 quel que soit l'orientation de l'embout par rapport à l'arbre 110.

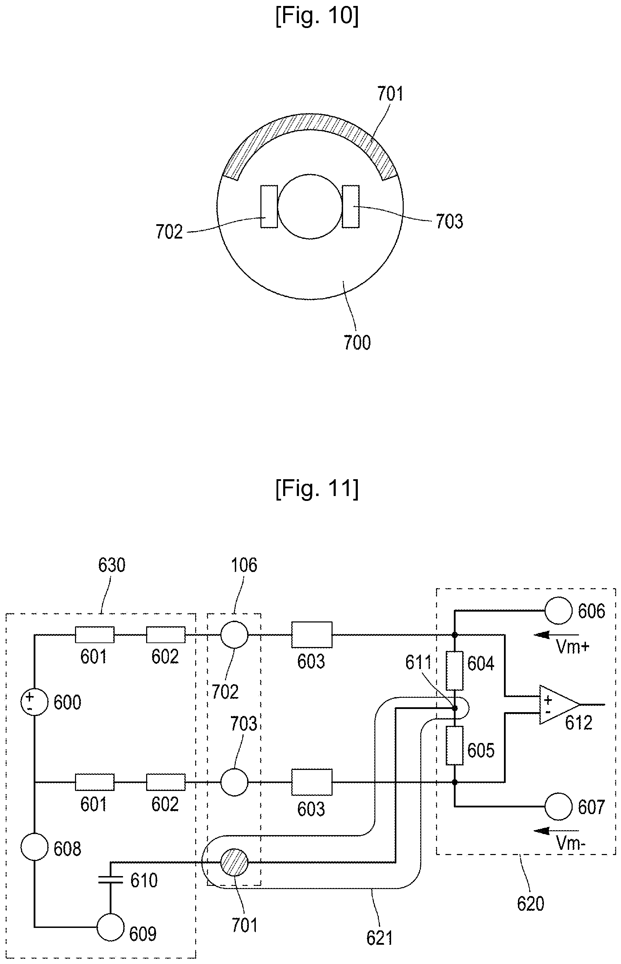

Les figures 9 et 10 représentent une réalisation d'un embout 700 avec trois électrodes pouvant être utilisé dans les dispositifs 100, 200 et 400 précités. Les électrodes 702 et 703, disposées de manière diamétralement opposée de part et d'autre de la partie cylindrique de l'embout, correspondent à une électrode de mesure et une électrode de référence. L'électrode 701, disposée plus en retrait par rapport aux deux autres, correspond à une électrode de masse. Sa position en retrait par rapport aux autres électrodes lui permet d'être en contact avec le tragus. Cela est permis par la forme bombée de l'embout dans la partie qui sera la plus proche de l'entrée du canal auditif. Ainsi, les trois électrodes 701, 702 et 703 peuvent être utilisées pour effectuer une mesure, comme il sera décrit ci-dessous ne référence la figure 11.

Il est possible d'effectuer plusieurs captations parallèles pour augmenter la précision de la mesure. Chaque captation supplémentaire requiert une électrode supplémentaire (qui sera une électrode de mesure). Pour effectuer deux captations, il est ainsi possible d'ajouter une électrode de mesure sur la partie cylindrique espacée dans une direction circonférentielle par rapport aux électrodes 702 et 703.

La figure 11 représente un schéma de principe du fonctionnement du dispositif muni de l'embout 700 lorsqu'il est en cours d'utilisation, au contact du corps 630 d'un mammifère vivant. On a représenté en particulier le circuit électrique permettant de mesurer une différence de potentiel entre le signal capté par une électrode de mesure et le signal capté par une électrode de référence. Les signaux proviennent du cerveau 600 du mammifère. Le mammifère peut par exemple être un être humain.

Sur la figure 11, le corps 630 est modélisée de la manière suivante. Le cerveau du mammifère 600 est modélisé comme un générateur de tension avec des résistances internes. La tension générée est de l'ordre de 100 µV. Les résistances internes ici sont constituées de la résistance de la tête de l'individu 601 et la résistance de la peau de l'individu 602. La résistance de la peau de l'individu 602 constitue la résistance la plus importante. Cette résistance varie entre 5 et 100 MΩ. Cette résistance dépend de plusieurs paramètres plus ou moins contrôlables. Le corps 630 comporte aussi des sources de bruit. Ces sources de bruit sont les générateurs électriques du corps (muscles du visage, yeux...), mais aussi des perturbations extérieures qui peuvent injecter du courant de temps en temps dans le circuit. Les générateurs de bruit internes sont modélisés par le générateur de bruit interne 608, et les sources de bruit externes à l'individu sont modélisées par le générateur de bruit externe 609. Le corps de l'individu agit alors comme une antenne, qui peut être modélisée par une capacité 610, de capacité variable selon le courant injecté par le générateur de bruit externe 609.

Afin de déterminer un état physiologique ou psychique de l'individu, il est nécessaire de mesurer une différence de potentiel dans le cerveau 600, soit ici entre les bornes positive et négative du générateur qui modélise le cerveau. Une électrode de mesure 702 capte un signal électrique au niveau de la borne positive et une électrode de référence 703 capte un signal électrique au niveau de la borne négative. Les tensions des deux signaux sont influencées par les résistances de la tête 601 et de la peau 602 de l'individu comme représenté sur le schéma. Chacun de ces signaux est acheminé vers un appareil de mesure 620 inclus dans les moyens de traitements 114 précités.

Avant d'arriver à l'appareil de mesure 620, chacun des deux signaux est amplifié en passant à travers un adaptateur d'impédance 603, qui permet une amplification de 10 à 12 fois. La tension du courant sortant de l'amplificateur est ainsi de l'ordre de 10 à 100 µV, alors que le signal initial a un potentiel de l'ordre du µV. Pour mesurer la tension du signal capté par l'électrode de mesure 702, respectivement par l'électrode de référence 703, l'appareil de mesure 620 comporte une résistance interne 604, respectivement 605, de valeur de résistance identique connue (en pratique, proche de 1 GΩ). Selon un mode de réalisation, la résistance peut être un ensemble de résistances montées en série La mesure est effectuée selon le principe d'un voltmètre. Cette résistance est branchée sur un générateur de tension 606 générant un potentiel Vm+, respectivement un générateur de tension 607 générant un potentiel Vm-. En pratique, une valeur choisie pour les potentiels est par exemple Vm+ = 2,4 V et Vm- = 0 V. Les signaux ainsi mesurés sont ensuite acheminés vers un amplificateur opérationnel 612. L'appareil de mesure 620 peut être réalisé au moyen d'un composant ADS 1292 disponible auprès de la société Texas Instrument.

Ce circuit permet de détecter les valeurs aberrantes lors de la mesure de la différence de potentiel entre les bornes + et - du générateur qui modélise le cerveau 600. En effet, la différence de potentiel mesurée doit alors fluctuer à 10 ou 100 µV près autour de 1,2V. Une différence de potentiel s'écartant significativement de cette valeur est alors déclarée comme étant aberrante. L'adaptateur d'impédance 603 permet aussi de limiter les mesures aberrantes. En effet, si l'un des signaux obtenus dépasse la fenêtre de valeurs comprise entre Vm- et Vm+, L'adaptateur d'impédance 603 n'amplifie pas le signal donc la mesure est directement détectée comme étant aberrante.

Au moment de calculer la différence de potentiel, il est également nécessaire d'enlever le bruit de la mesure. Dans le mode de réalisation représenté sur la figure 11, l'électrode de masse 701 est utilisée pour supprimer le bruit provenant des sources de bruit extérieures à l'individu représentées par le générateur 609. La solution pour supprimer le bruit est un circuit de réjection de mode commun 621. Pour cela, l'électrode de masse 701 est reliée au point milieu 611 entre les résistances 604 et 605.

Dans la modélisation adoptée, l'effet de ce branchement est de court-circuiter la capacité 610 en injectant dans le circuit un courant identique à celui généré par le générateur de bruit externe 609. Ce courant correspond au signal capté par l'électrode de masse 701 en contact avec une zone du canal auditif très stable, dans laquelle les variations de signal électrique correspondent aux variations de bruit extérieur. Une telle zone stable qui permet de capter le bruit extérieur le plus pur possible est par exemple le tragus, un cartilage situé dans l'oreille à l'entrée du canal auditif. En pratique, le circuit de réjection de mode commun 621 est mis en œuvre en branchant l'électrode de masse 701 sur un potentiel correspondant à la demie-somme de Vm+ et Vm-, afin que le potentiel nul soit défini de la même manière dans tout le circuit. Sur la figure 11, c'est le point milieu 611 qui correspond à ce potentiel.

En effet, il est nécessaire que le potentiel nul correspondant au courant permettant de faire la réduction de bruit soit le même potentiel autour duquel fluctue la différence de potentiel mesurée. Avec les valeurs données précédemment, la différence de potentiel fluctue autour de 1,2V donc il faut que l'électrode de masse 701 soit branchée sur ce potentiel de 1,2V.

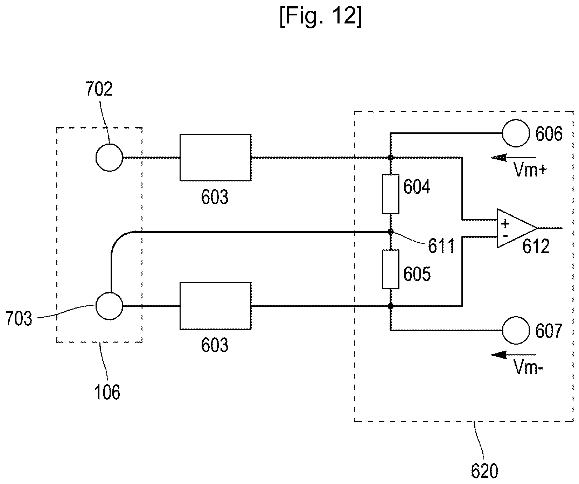

Dans le mode de réalisation de la figure 12, la pluralité d'électrodes 106 ne comporte qu'une électrode de mesure 702 et une électrode de référence 703. L'électrode de masse 701 est supprimée. Le bruit provenant du générateur 609 est supprimé en branchant l'électrode de référence 703 au point milieu 611 correspondant à la demie-somme de Vm+ et Vm-. Dans ce mode de réalisation, tout se passe comme s'il y avait une électrode de masse virtuelle confondue avec l'électrode de référence 703.

Les applications d'un tel dispositif 100, 200, ou 400 sont nombreuses, notamment en médecine, notamment le suivi de patients atteints de maladies neuronales, dont l'épilepsie, le dépistage et le diagnostic de maladies neuronales, le dépistage et le suivi d'enfants atteints de troubles de l'attention, mais aussi pour le suivi de certains professionnels, notamment le suivi des contrôleurs aériens ou des pilotes dans l'aviations, le suivi des sportifs, ou encore pour améliorer la vie quotidienne par exemple l'élaboration d'applications de bien-être.

Bien que l'invention ait été décrite en liaison avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention défini par les revendications.

L'usage du verbe « comporter », « comprendre » ou « inclure » et de ses formes conjuguées n'exclut pas la présence d'autres éléments ou d'autres étapes que ceux énoncés dans une revendication.

Dans les revendications, tout signe de référence entre parenthèses ne saurait être interprété comme une limitation de la revendication.

## Revendications

1. Dispositif (100,200,400) pour déterminer un état physiologique ou psychique d'un mammifère, le dispositif (100,200,400) comprenant au moins une oreillette (402) comprenant :
- un corps principal (102) comprenant un corps de révolution (110) ayant un axe de révolution (108), et
- un embout (104,220,700) configuré pour être inséré dans un canal auditif (404), ledit embout (104, 220) présentant un canal cylindrique (112) destiné à recevoir le corps de révolution (110) pour monter l'embout (104, 220) sur le corps principal (102) de manière amovible, l'embout (104, 220) étant agencé déplaçable en rotation autour de l'axe de révolution (108) de sorte à permettre d'orienter au moins une électrode (106) vers une zone du cerveau du mammifère, et ledit embout (104, 220) comprenant une pluralité d'électrodes (106 ; 701-703) agencées sur une surface extérieure de l'embout (104, 220), la pluralité d'électrodes (106) comportant une électrode de référence (703) et au moins une électrode de mesure (702), chaque électrode (106 ; 701-703) étant configurée pour capter un signal électrique dans le canal auditif du mammifère, et
ledit dispositif comprenant des premières pistes électriques (118,202) isolées électriquement l'une de l'autre, agencées dans le canal cylindrique (112) de l'embout (104,220) et reliées aux électrodes (106) et des deuxièmes pistes électriques (116, 204) isolées électriquement l'une de l'autre, agencées dans la partie cylindrique (110) du corps principal (102) et destinées à acheminer le signal électrique capté par les électrodes (106) vers un moyen de calcul (115),
dans lequel chacune des premières pistes électriques, ou chacune des deuxièmes pistes électriques, présente une forme annulaire ayant pour axe ledit axe de révolution et est agencée en contact électrique avec une desdites premières pistes électriques (118,202), ou une desdites deuxièmes pistes électriques, indépendamment de l'orientation de l'embout autour dudit axe de révolution, et lesdites premières (118,202) ou deuxièmes pistes électriques (116,204) de forme annulaire étant espacées le long dudit axe de révolution (108),
**caractérisé par le fait que** le dispositif comporte en outre des moyens de traitement (114, 620) incluant deux générateurs de tension (606, 607), l'électrode de référence (703) étant branchée sur un potentiel correspondant à une demie-somme des tensions délivrées par les deux générateurs de tension (606, 607),
le dispositif comportant ledit moyen de calcul (115) configuré pour déterminer un état physiologique ou psychique d'un mammifère en fonction d'une différence entre le signal électrique capté par l'électrode de mesure (702) et le signal électrique capté par l'électrode de référence (703).

2. Dispositif (100, 200, 400) selon la revendication 1, dans lequel l'électrode de référence est destinée à être orientée vers une mastoïde du mammifère lors de l'insertion de l'embout dans le canal auditif du mammifère.

3. Dispositif (100, 200, 400) selon la revendication 1 ou 2, dans lequel l'embout (104, 220) comporte un marquage configuré pour reconnaître visuellement l'électrode de référence.

4. Dispositif (100, 200, 400) selon l'une des revendications 1 à 3, dans lequel les électrodes (106, 702, 703) sont espacées mutuellement dans une direction circonférentielle autour de l'axe de révolution (108).

5. Dispositif (400) selon la revendication 1, dans lequel le dispositif (400) comprend deux oreillettes (402, 402') comportant respectivement l'électrode de mesure (702) et l'électrode de référence (703) et configurées pour être agencées respectivement dans un premier canal (404) auditif du mammifère et un deuxième canal (404') auditif du mammifère et destinées à acheminer des signaux électriques respectifs au moyen de calcul (115),
ledit moyen de calcul (115) étant apte pour déterminer l'état physiologique ou psychique en fonction du signal électrique capté par l'électrode de mesure (702) de l'oreillette (402) agencée dans le premier canal auditif (404) et du signal électrique capté par l'électrode de référence de l'oreillette (402') agencée dans le deuxième canal auditif (404').

6. Dispositif (100, 200, 400) pour déterminer un état physiologique ou psychique d'un mammifère, le dispositif (100,200,400) comprenant au moins une oreillette (402) comprenant :
- un corps principal (102) comprenant un corps de révolution (110) ayant un axe de révolution (108), et
- un embout (104,220,700) configuré pour être inséré dans un canal auditif (404), ledit embout (104, 220) présentant un canal cylindrique (112) destiné à recevoir le corps de révolution (110) pour monter l'embout (104, 220) sur le corps principal (102) de manière amovible, l'embout (104, 220) étant agencé déplaçable en rotation autour de l'axe de révolution (108) de sorte à permettre d'orienter au moins une électrode (106) vers une zone du cerveau du mammifère, et ledit embout (104, 220) comprenant une pluralité d'électrodes (106 ; 701-703) agencées sur une surface extérieure de l'embout (104, 220), la pluralité d'électrodes (106) comportant une électrode de référence (703), au moins une électrode de mesure (702) et une électrode de masse (701), ladite électrode de masse étant orientée vers une partie spécifique du canal auditif, ladite partie spécifique du canal auditif permettant de capter un signal stable, chaque électrode (106 ; 701-703) étant configurée pour capter un signal électrique dans le canal auditif du mammifère, et
ledit dispositif comprenant des premières pistes électriques (118,202) isolées électriquement l'une de l'autre, agencées dans le canal cylindrique (112) de l'embout (104,220) et reliées aux électrodes (106) et des deuxièmes pistes électriques (116, 204) isolées électriquement l'une de l'autre, agencées dans la partie cylindrique (110) du corps principal (102) et destinées à acheminer le signal électrique capté par les électrodes (106) vers un moyen de calcul (115),
dans lequel chacune des premières pistes électriques, ou chacune des deuxièmes pistes électriques, présente une forme annulaire ayant pour axe ledit axe de révolution et est agencée en contact électrique avec une desdites premières pistes électriques (118,202), ou une desdites deuxièmes pistes électriques, indépendamment de l'orientation de l'embout autour dudit axe de révolution, et lesdites premières (118,202) ou deuxièmes pistes électriques (116,204) de forme annulaire étant espacées le long dudit axe de révolution (108),
**caractérisé par le fait que** le dispositif comporte des moyens de traitement (114, 620) configurés pour utiliser le signal capté par l'électrode de masse (701) pour effectuer une réduction de bruit dans les signaux mesurés par l'électrode de référence (703) et l'au moins une électrode de mesure (702), les moyens de traitement comportant deux générateurs de tension (606, 607) et l'électrode de masse (701) étant branchée sur un potentiel correspondant à une demie-somme des tensions délivrées par les deux générateurs de tension (606, 607),
le dispositif comportant un moyen de calcul (115) configuré pour déterminer un état physiologique ou psychique d'un mammifère en fonction d'une différence entre le signal électrique capté par l'électrode de mesure (702) et le signal électrique capté par l'électrode de référence (703).

7. Dispositif (100, 200, 400) selon la revendication 6, dans lequel l'embout (104, 220, 700) est constitué de sorte à pouvoir être en contact avec la partie spécifique du canal auditif lorsqu'il est inséré dans le canal auditif, et dans lequel l'électrode de masse (701) est située sur une partie de l'embout (104, 220, 700) en contact avec la partie spécifique du canal auditif afin de de capter un signal électrique sur ladite partie spécifique du canal auditif.

8. Dispositif (100, 200, 400) selon l'une des revendications 6 à 7, dans laquelle la partie spécifique du canal auditif est un tragus du mammifère.

9. Dispositif (100, 200, 400) selon la revendication 6, dans lequel les moyens de traitement comportent une première résistance (605) branchée sur un premier générateur de tension (607) et agencée de manière à recevoir le signal électrique capté par l'électrode de référence et une deuxième résistance (604) branchée sur un deuxième générateur de tension (606) et agencée de manière à recevoir le signal électrique capté par l'au moins une électrode de mesure, le deuxième générateur de tension délivrant une tension supérieure à celle délivrée par le premier générateur de tension.

10. Dispositif (100, 200, 400) selon l'une des revendications 1 à 9, comprenant un sélectionneur configuré pour recevoir les signaux électriques des électrodes, ledit sélectionneur étant configuré pour détecter une électrode de référence, parmi les électrodes (106), ladite électrode de référence étant sélectionnée en tant que l'électrode émettant le signal électrique ayant le potentiel électrique le plus stable parmi les signaux électriques captés par les électrodes (106).

11. Dispositif (100, 200, 400) selon l'une quelconque des revendications 1 à 10, dans lequel le corps principal (102) comporte un moyen de transmission (308) de données filaire ou sans fil configuré pour communiquer avec le moyen de calcul (115).

12. Dispositif (100, 200, 400) selon l'une quelconque des revendications 1 à 11, dans lequel le dispositif comprend un moyen de filtrage des signaux électriques configuré pour atténuer des signaux parasites détectés par une ou plusieurs desdites électrodes, le moyen de filtrage comprenant un filtre passe bande (306) ayant une bande passante comprise entre 0,3 Hz et 50 Hz, en particulier comprise entre 1 Hz et 40 Hz

13. Dispositif (100, 200, 400) selon l'une quelconque des revendications 1 à 12, dans lequel le moyen de calcul est configuré pour :
déterminer un signal d'électroencéphalogramme du mammifère en fonction des signaux électriques mesurés,
déterminer une amplitude d'au moins une onde cérébrale dans une plage de fréquence prédéfinie en fonction dudit signal d'électroencéphalogramme, et
déterminer un état psychique en fonction de ladite amplitude d'au moins une onde cérébrale par comparaison de ladite amplitude à un seuil prédéterminé.

14. Dispositif (100,200,400) selon l'une quelconque des revendications 1 à 13, dans lequel l'état physiologique ou psychique est relatif à une activité électrique d'un organe du mammifère sélectionné dans le groupe constitué d'un cerveau, un coeur, un nerf, un muscle et un œil.

15. Dispositif (100,200,400) selon l'une quelconque des revendications 1 à 14, dans lequel l'état physiologique ou psychique est une fatigue du mammifère, un niveau d'attention du mammifère ou une crise d'épilepsie du mammifère.

## Patentansprüche

1. Vorrichtung (100, 200, 400) zur Bestimmung eines physiologischen oder psychischen Zustands eines Säugetiers, wobei die Vorrichtung (100, 200, 400) mindestens ein Ohrstück (402) umfasst, das Folgendes umfasst:
- einen Hauptkörper (102), der einen Rotationskörper (110) umfasst, der eine Rotationsachse (108) aufweist, und
- einen Aufsatz (104, 220, 700), der so konfiguriert ist, dass er in einen Gehörgang (404) eingeführt werden kann, wobei der Aufsatz (104, 220) einen zylindrischen Kanal (112) aufweist, der dazu bestimmt ist, den Rotationskörper (110) aufzunehmen, um den Aufsatz (104, 220) abnehmbar am Hauptkörper (102) zu befestigen, wobei der Aufsatz (104, 220) um die Rotationsachse (108) drehbar angeordnet ist, um mindestens eine Elektrode (106) auf einen Bereich des Gehirns des Säugetiers auszurichten, und wobei der Aufsatz (104, 220) eine Vielzahl von Elektroden umfasst, die an einer Außenfläche des Aufsatzes (104, 220) angeordnet sind, wobei die Vielzahl von Elektroden (106) eine Referenzelektrode (703) und mindestens eine Messelektrode (702) umfasst, wobei jede Elektrode (106; 701-703) so konfiguriert ist, dass sie ein elektrisches Signal im Gehörgang des Säugetiers erfasst, und
wobei die Vorrichtung erste elektrische Bahnen (118, 202) umfasst, die elektrisch voneinander isoliert sind, im zylindrischen Kanal (112) des Ohrstücks (104, 220) angeordnet sind und mit den Elektroden (106) verbunden sind, und zweite elektrische Bahnen (116, 204), die elektrisch voneinander isoliert sind, im zylindrischen Teil (110) des Hauptkörpers (102) angeordnet sind und dazu bestimmt sind, das von den Elektroden (106) erfasste elektrische Signal mit Hilfe einer Recheneinrichtung (115) an einen Ort zu leiten, wobei jede der ersten elektrischen Bahnen oder jede der zweiten elektrischen Bahnen eine ringförmige Gestalt aufweist, deren Achse die Rotationsachse ist, und unabhängig von der Ausrichtung des Aufsatzes um die genannte Rotationsachse in elektrischem Kontakt mit einer der genannten ersten elektrischen Bahnen (118, 202) oder einer der genannten zweiten elektrischen Bahnen angeordnet ist, und wobei die ringförmigen ersten (118, 202) oder zweiten elektrischen Bahnen (116, 204) entlang der Rotationsachse (108) beabstandet sind,
**dadurch gekennzeichnet, dass** die Vorrichtung weiterhin Verarbeitungsmittel (114, 620) umfasst, die zwei Spannungsgeneratoren (606, 607) enthalten, wobei die Referenzelektrode (703) an ein Potential angeschlossen ist, das der halben Summe der von den beiden Spannungsgeneratoren (606, 607) gelieferten Spannungen entspricht,
wobei die Vorrichtung die Recheneinrichtung (115) umfasst, die so konfiguriert ist, dass sie einen physiologischen oder psychischen Zustand eines Säugetiers in Abhängigkeit von einer Differenz zwischen dem von der Messelektrode (702) erfassten elektrischen Signal und dem von der Referenzelektrode (703) erfassten elektrischen Signal bestimmt.

2. Vorrichtung (100, 200, 400) gemäß Anspruch 1, wobei die Referenzelektrode dazu bestimmt ist, beim Einführen des Aufsatzes in den Gehörgang des Säugetiers auf dessen Mastoid ausgerichtet zu werden.

3. Vorrichtung (100, 200, 400) gemäß Anspruch 1 oder 2, wobei der Aufsatz (104, 220) eine Markierung umfasst, die so gestaltet ist, dass die Referenzelektrode visuell erkennbar ist.

4. Vorrichtung (100, 200, 400) gemäß einem der Ansprüche 1 bis 3, wobei die Elektroden (106, 702, 703) in Umfangsrichtung um die Rotationsachse (108) voneinander beabstandet sind.

5. Vorrichtung (400) gemäß Anspruch 1, wobei die Vorrichtung (400) zwei Ohrstücke (402, 402') umfasst, die jeweils die Messelektrode (702) und die Referenzelektrode (703) umfassen und konfiguriert sind, um jeweils in einem ersten Gehörgang (404) des Säugetiers und einem zweiten Gehörgang (404') des Säugetiers anordnenbar zu sein und dazu bestimmt sind, jeweilige elektrische Signale mit Hilfe der Recheneinrichtung (115) an diese zu leiten, wobei die Recheneinrichtung (115) in der Lage ist, den physiologischen oder psychischen Zustand in Abhängigkeit von dem von der Messelektrode (702) des im ersten Gehörgang (404) angeordneten Ohrstücks (402) und des von der Referenzelektrode des im zweiten Gehörgang (404') angeordneten Ohrstücks (402') erfassten elektrischen Signals zu bestimmen.

6. Vorrichtung (100, 200, 400) zur Bestimmung eines physiologischen oder psychischen Zustands eines Säugetiers, wobei die Vorrichtung (100, 200, 400) mindestens ein Ohrstück (402) umfasst, das Folgendes umfasst:
- einen Hauptkörper (102), der einen Rotationskörper (110) umfasst, der eine Rotationsachse (108) aufweist, und
- einen Aufsatz (104, 220, 700), der so konfiguriert ist, dass er in einen Gehörgang (404) eingeführt werden kann, wobei der Aufsatz (104, 220) einen zylindrischen Kanal (112) aufweist, der dazu bestimmt ist, den Rotationskörper (110) aufzunehmen, um den Aufsatz (104, 220) abnehmbar am Hauptkörper (102) zu befestigen, wobei der Aufsatz (104, 220) drehbar um die Rotationsachse (108) angeordnet ist, um mindestens eine Elektrode (106) auf einen Bereich des Gehirns des Säugetiers auszurichten, und wobei der Aufsatz (104, 220) eine Vielzahl von Elektroden umfasst (106; 701-703) , die an einer Außenfläche des Aufsatzes (104, 220) angeordnet sind, wobei die Vielzahl von Elektroden (106) eine Referenzelektrode (703), mindestens eine Messelektrode (702) und eine Masseelektrode (701) umfasst, wobei die Masseelektrode auf einen bestimmten Abschnitt des Gehörgangs ausgerichtet ist, wobei der bestimmte Abschnitt des Gehörgangs die Erfassung eines stabilen Signals ermöglicht, wobei jede Elektrode (106; 701-703) so konfiguriert ist, dass sie ein elektrisches Signal im Gehörgang des Säugetiers erfasst, und wobei die Vorrichtung erste elektrische Bahnen (118, 202) umfasst, die elektrisch voneinander isoliert sind, im zylindrischen Kanal (112) des Ohrstücks (104, 220) angeordnet sind und mit den Elektroden (106) verbunden sind, und zweite elektrische Bahnen (116, 204), die elektrisch voneinander isoliert sind, im zylindrischen Teil (110) des Hauptkörpers (102) angeordnet sind und dazu bestimmt sind, das von den Elektroden (106) erfasste elektrische Signal mit Hilfe einer Recheneinrichtung (115) zu leiten,
wobei jede der ersten elektrischen Bahnen oder jede der zweiten elektrischen Bahnen eine ringförmige Gestalt aufweist, deren Achse die genannte Rotationsachse ist, und unabhängig von der Ausrichtung des Aufsatzes um die genannte Rotationsachse in elektrischem Kontakt mit einer der genannten ersten elektrischen Bahnen (118, 202) oder einer der genannten zweiten elektrischen Bahnen angeordnet ist, und wobei die ringförmigen ersten (118, 202) oder zweiten elektrischen Bahnen (116, 204) entlang der Drehachse (108) beabstandet sind,
**dadurch gekennzeichnet, dass** die Vorrichtung Verarbeitungsmittel (114, 620) umfasst, die so konfiguriert sind, dass sie das von der Masseelektrode (701) erfasste Signal nutzen, um eine Rauschunterdrückung in den von der Referenzelektrode (703) und der mindestens einen Messelektrode (702) gemessenen Signalen durchzuführen, wobei die Verarbeitungsmittel zwei Spannungsgeneratoren (606, 607) umfassen und die Masseelektrode (701) an ein Potential angeschlossen ist, das der halben Summe der von den beiden Spannungsgeneratoren (606, 607) gelieferten Spannungen entspricht,
wobei die Vorrichtung eine Recheneinheit (115) umfasst, die so konfiguriert ist, dass sie einen physiologischen oder psychischen Zustand eines Säugetiers in Abhängigkeit von einer Differenz zwischen dem von der Messelektrode (702) erfassten elektrischen Signal und dem von der Referenzelektrode (703) erfassten elektrischen Signal bestimmt.

7. Vorrichtung (100, 200, 400) gemäß Anspruch 6, wobei der Aufsatz (104, 220, 700) so ausgebildet ist, dass es bei Einführen in den Gehörgang mit dem spezifischen Abschnitt des Gehörgangs in Kontakt kommen kann, und wobei sich die Masseelektrode (701) an einem Abschnitt des Ohrstücks (104, 220, 700) befindet, der mit dem spezifischen Teil des Gehörgangs in Kontakt steht, um ein elektrisches Signal an diesem spezifischen Teil des Gehörgangs zu erfassen.

8. Vorrichtung (100, 200, 400) gemäß einem der Ansprüche 6 bis 7, wobei der spezifische Teil des Gehörgangs ein Tragus des Säugetiers ist.

9. Vorrichtung (100, 200, 400) gemäß Anspruch 6, wobei die Verarbeitungsmittel einen ersten Widerstand (605) umfassen, der an einen ersten Spannungsgenerator (607) angeschlossen und so angeordnet ist, dass er das von der Referenzelektrode erfasste elektrische Signal empfängt, sowie einen zweiten Widerstand (604), der an einen zweiten Spannungsgenerator (606) angeschlossen und so angeordnet ist, dass er das von der mindestens einen Messelektrode erfasste elektrische Signal empfängt, wobei der zweite Spannungsgenerator eine höhere Spannung liefert als der erste Spannungsgenerator.

10. Vorrichtung (100, 200, 400) gemäß einem der Ansprüche 1 bis 9, umfassend einen Selektor, der so konfiguriert ist, dass er die elektrischen Signale der Elektroden empfängt, wobei der Selektor so konfiguriert ist, dass er unter den Elektroden (106) eine Referenzelektrode erkennt, wobei die Referenzelektrode als diejenige Elektrode ausgewählt wird, die das elektrische Signal mit dem stabilsten elektrischen Potential unter den von den Elektroden (106) erfassten elektrischen Signalen aussendet.

11. Vorrichtung (100, 200, 400) gemäß einem der Ansprüche 1 bis 10, wobei der Hauptkörper (102) eine drahtgebundene oder drahtlose Datenübertragungseinrichtung (308) umfasst, die so konfiguriert ist, dass sie mit der Recheneinrichtung (115) kommuniziert.

12. Vorrichtung (100, 200, 400) gemäß einem der Ansprüche 1 bis 11, wobei die Vorrichtung ein Mittel zum Filtern elektrischer Signale umfasst, das so konfiguriert ist, dass es Störsignale dämpft, die von einer oder mehreren der genannten Elektroden erfasst werden, wobei die Filtervorrichtung einen Bandpassfilter (306) umfasst, der eine Bandbreite zwischen 0,3 Hz und 50 Hz, vor allem zwischen 1 Hz und 40 Hz, aufweist.

13. Vorrichtung (100, 200, 400) gemäß einem der Ansprüche 1 bis 12, wobei die Recheneinrichtung so konfiguriert ist, dass sie:
ein Elektroenzephalogramm-Signal des Säugetiers auf der Grundlage der gemessenen elektrischen Signale bestimmt,
eine Amplitude mindestens einer Gehirnwelle in einem vordefinierten Frequenzbereich auf der Grundlage des Elektroenzephalogramm-Signals bestimmt und
einen psychischen Zustand auf der Grundlage der Amplitude mindestens einer Gehirnwelle durch Vergleich dieser Amplitude mit einem vorgegebenen Schwellenwert bestimmt.

14. Vorrichtung (100, 200, 400) gemäß einem der Ansprüche 1 bis 13, wobei sich der physiologische oder psychische Zustand auf eine elektrische Aktivität eines Organs des Säugetiers bezieht, das aus der Gruppe ausgewählt ist, die aus einem Gehirn, einem Herzen, einem Nerv, einem Muskel und einem Auge besteht.

15. Vorrichtung (100, 200, 400) gemäß einem der Ansprüche 1 bis 14, wobei der physiologische oder psychische Zustand eine Ermüdung des Säugetiers, ein Aufmerksamkeitsniveau des Säugetiers oder ein epileptischer Anfall des Säugetiers ist.

## Claims

1. A device (100, 200, 400) for determining a physiological or psychological state of a mammal, the device (100, 200, 400) comprising at least one earpiece (402) comprising:
- a main body (102) comprising a body of revolution (110) having an axis of revolution (108), and
- an endpiece (104, 220, 700) configured to be inserted into an ear canal (404), said endpiece (104, 220) having a cylindrical channel (112) intended to receive the body of revolution (110) for detachably mounting the endpiece (104, 220) on the main body (102), the endpiece (104, 220) being arranged to be movable in rotation about the axis of revolution (108) in order to allow at least one electrode (106) to be oriented toward a zone of the brain of the mammal, and said endpiece (104, 220) comprising a plurality of electrodes (106; 701-703) arranged on an outer surface of the endpiece (104, 220), the plurality of electrodes (106) comprising a reference electrode (703) and at least one measuring electrode (702), each electrode (106; 701-703) being configured to pick up an electrical signal in the ear canal of the mammal, and
said device comprising first electrical tracks (118, 202) electrically insulated from each other, arranged in the cylindrical channel (112) of the endpiece (104, 220) and connected to the electrodes (106), and second electrical tracks (116, 204) electrically insulated from each other, arranged in the cylindrical part (110) of the main body (102) and intended to convey the electrical signal, picked up by the electrodes (106), to a calculation means (115),
in which each of the first electrical tracks, or each of the second electrical tracks, has an annular shape having as its axis said axis of revolution and is arranged in electrical contact with one of said first electrical tracks (118, 202), or one of said second electrical tracks, independently of the orientation of the endpiece about said axis of revolution, and said first electrical tracks (118, 202) or second electrical tracks (116, 204) of annular shape being spaced along said axis of revolution (108)
**characterized in that** the device further comprises processing means (114, 620) including two voltage generators (606, 607), in which the reference electrode (703) is connected to a potential corresponding to half the sum of the voltages delivered by the two voltage generators (606, 607).
the device comprising the calculation means (115) configured to determine a physiological or psychological state of a mammal as a function of a difference between the electrical signal picked up by the measuring electrode (702) and the electrical signal picked up by the reference electrode (703).

2. The device (100, 200, 400) as claimed in claim 1, in which the reference electrode is intended to be oriented toward a mastoid of the mammal upon insertion of the endpiece into the ear canal of the mammal.

3. The device (100, 200, 400) as claimed in claim 1 or 2, in which the endpiece (104, 220) has a marking configured for visually recognizing the reference electrode.

4. The device (100, 200, 400) as claimed in any one of claims 1 to 3, in which the electrodes (106, 702, 703) are spaced apart from each other in a circumferential direction about the axis of revolution (108).

5. The device (400) as claimed in claim 1, in which the device (400) comprises two earpieces (402, 402') respectively including the measuring electrode (702) and the reference electrode (703) and configured to be arranged in a first mammalian ear canal (404) and a second mammalian ear canal (404'), respectively, and intended to convey respective electrical signals to the calculation means (115), said calculation means (115) being suitable for determining the physiological or psychological state as a function of the electrical signal picked up by the measuring electrode (702) of the earpiece (402) arranged in the first ear canal (404) and the electrical signal picked up by the reference electrode (703) of the earpiece (402') arranged in the second ear canal (404'), in particular as a function of a difference between the two signals.

6. A device (100, 200, 400) for determining a physiological or psychological state of a mammal, the device (100, 200, 400) comprising at least one earpiece (402) comprising:
- a main body (102) comprising a body of revolution (110) having an axis of revolution (108), and
- an endpiece (104, 220, 700) configured to be inserted into an ear canal (404), said endpiece (104, 220) having a cylindrical channel (112) intended to receive the body of revolution (110) for detachably mounting the endpiece (104, 220) on the main body (102), the endpiece (104, 220) being arranged to be movable in rotation about the axis of revolution (108) in order to allow at least one electrode (106) to be oriented toward a zone of the brain of the mammal, and said endpiece (104, 220) comprising a plurality of electrodes (106; 701-703) arranged on an outer surface of the endpiece (104, 220), the plurality of electrodes (106) comprising a reference electrode (703), at least one measuring electrode (702) and a ground electrode (701), said ground electrode being oriented toward a specific part of the ear canal, said specific part of the ear canal making it possible to pick up a stable signal, each electrode (106; 701-703) being configured to pick up an electrical signal in the ear canal of the mammal, and said device comprising first electrical tracks (118, 202) electrically insulated from each other, arranged in the cylindrical channel (112) of the endpiece (104, 220) and connected to the electrodes (106), and second electrical tracks (116, 204) electrically insulated from each other, arranged in the cylindrical part (110) of the main body (102) and intended to convey the electrical signal, picked up by the electrodes (106), to a calculation means (115),
in which each of the first electrical tracks, or each of the second electrical tracks, has an annular shape having as its axis said axis of revolution and is arranged in electrical contact with one of said first electrical tracks (118, 202), or one of said second electrical tracks, independently of the orientation of the endpiece about said axis of revolution, and said first electrical tracks (118, 202) or second electrical tracks (116, 204) of annular shape being spaced along said axis of revolution (108)
**characterized in that** the device comprises processing means (114, 620) configured to use the signal picked up by the ground electrode (701) to effect a noise reduction in the signals measured by the reference electrode (703) and the at least one measuring electrode (702), the processing means comprising two voltage generators (606, 607), wherein the ground electrode (701) is connected to a potential corresponding to half the sum of the voltages delivered by the two voltage generators (606, 607),
the device comprising the calculation means (115) configured to determine a physiological or psychological state of a mammal as a function of a difference between the electrical signal picked up by the measuring electrode (702) and the electrical signal picked up by the reference electrode (703).

7. The device (100, 200, 400) as claimed in claim 6, in which the endpiece (104, 220, 700) is constructed so as to be able to be in contact with the specific part of the ear canal when inserted into the ear canal, and in which the ground electrode (701) is located on a part of the endpiece (104, 220, 700) in contact with the specific part of the ear canal, in order to pick up an electrical signal on said specific part of the ear canal.

8. The device (100, 200, 400) as claimed in either of claims 6 and 7, in which the specific part of the ear canal is a mammalian tragus.

9. The device (100, 200, 400) as claimed in claim 6, in which the processing means comprise a first resistor (605) connected to a first voltage generator (607) and arranged in such a way as to receive the electrical signal picked up by the reference electrode, and a second resistor (604) connected to a second voltage generator (606) and arranged in such a way as to receive the electrical signal picked up by the at least one measuring electrode, the second voltage generator delivering a voltage greater than that delivered by the first voltage generator.

10. The device (100, 200, 400) as claimed in one of claims 1 through 9, comprising a selector configured to receive the electrical signals from the electrodes, said selector being configured to detect a reference electrode, among the electrodes (106), said reference electrode being selected as the electrode emitting the electrical signal having the most stable electrical potential among the electrical signals picked up by the electrodes (106).

11. The device (100, 200, 400) as claimed in any one of claims 1 through 10, in which the main body (102) comprises wired or wireless data transmission means (308) configured to communicate with the calculation means (115).

12. The device (100, 200, 400) as claimed in any one of claims 1 through 11, in which the device comprises a means for filtering the electrical signals, said means being configured to attenuate parasitic signals detected by one or more of said electrodes, in which the filtering means comprises a band-pass filter (306) having a pass band between 0.3 Hz and 50 Hz, in particular between 1 Hz and 40 Hz.

13. The device (100, 200, 400) as claimed in any one of claims 1 through 12, in which the calculation means is configured to:
determine an electroencephalogram signal of the mammal as a function of the electrical signals measured,
determine an amplitude of at least one brain wave in a predefined frequency range as a function of said electroencephalogram signal, and
determine a psychological state as a function of said amplitude of at least one brain wave by comparing said amplitude with a predetermined threshold.

14. The device (100, 200, 400) as claimed in any one of claims 1 through 13, in which the physiological or psychological state relates to an electrical activity of an organ of the mammal, selected from the group consisting of a brain, a heart, a nerve, a muscle and an eye.

15. The device (100, 200, 400) as claimed in any one of claims 1 through 14, in which the physiological or psychological state is mammalian fatigue, a mammalian attention level or a mammalian epileptic seizure.
